# EUROPEAN PATENT SPECIFICATION

(11) **EP 2 180 029 B1**
(45) Date of publication and mention of the grant of the patent: **27.07.2011**
(21) Application number: 08019300.6
(22) Date of filing: 04.11.2008
(51) Int. Cl.: C09K 11/06, H01L 51/00

(54) **Radialene compounds and their use**
Radialenverbindungen und deren Verwendung
Composés de Radialène et leur utilisation

(30) Priority: 23.10.2008 EP 08018557
(43) Date of publication of application: 28.04.2010
(73) Proprietor: Novaled AG, 01307 Dresden (DE)
(72) Inventor: Zeika, Olaf, 06727 THEISSEN (DE); Willmann, Steffen, 01277 Dresden (DE); Bachmann, Christine, 01445 Radebeul (DE); Dorok, Sascha, 01067 Dresden (DE); Werner, Ansgar, 01277 Dresden (DE)
(74) Representative: Scholz, Volker

(56) References cited:
- KAZUKO TAKAHASHI ET AL: "NOVEL METALLIC CHARGE-TRANSFER COMPLEXES COMPOSED OF A Ú3 3/4 RADIALENETYPE ACCEPTOR: A 1,2-BIS(P-BENZOQUINO)-3-Ú2-(DICYANOMETHYLE NE- 2,5-SELENOQUINO 3/4 CYCLOPROPANE DERIVATIVE" ADVANCED MATERIALS, WILEY VCH, WEINHEIM, DE, vol. 7, no. 7, 1 July 1995 (1995-07-01), pages 639-641, XP000520477 ISSN: 0935-9648
- IYODA M ET AL: "Novel synthesis of hexaaryl[3]radialenes via dibromo[3]dendralenes" TETRAHEDRON LETTERS, ELSEVIER, AMSTERDAM, vol. 41, no. 36, 1 September 2000 (2000-09-01), pages 7059-7064, XP004208260 ISSN: 0040-4039
- DESPOTOVIC ET AL: "The structure and stability of [3]-radialenes and their dianions - A DFT study" JOURNAL OF MOLECULAR STRUCTURE (THEOCHEM), ELSEVIER SCIENCE PUBLISHERS B.V., AMSTERDAM, NL, vol. 811, no. 1-3, 11 May 2007 (2007-05-11), pages 313-322, XP022070531 ISSN: 0166-1280
- ENOMOTO T ET AL: "Hexaaryl[3]radialenes" TETRAHEDRON LETTERS, ELSEVIER, AMSTERDAM, vol. 38, no. 15, 14 April 1997 (1997-04-14), pages 2693-2696, XP004058306 ISSN: 0040-4039
- AUMANN R ET AL: "Organic syntheses via transition metal complexes. LXXXI. Bis(carbene) complex of chromium connected by a conjugated ammonium pentadienide bridge" JOURNAL OF ORGANOMETALLIC CHEMISTRY, ELSEVIER-SEQUOIA S.A. LAUSANNE, CH, vol. 502, no. 1, 18 October 1995 (1995-10-18), pages 137-141, XP004023719 ISSN: 0022-328X

## Description

The present invention relates to radialene compounds as well as to their use as organic doping agent for doping an organic semiconductive matrix material for changing its electrical properties, as blocker material as well as charge injection layer and as electrode material. The invention also relates to organic semiconductive materials as well as to electronic components in which the radialene compounds are used.

In the present application alicyclics in which all ring atoms are sp2-hybridized and to the extent possible carry exocyclic C-C double bonds are designated as radialenes, see also H. Hopf and G. Maas, Angew. Chem. (1992), 8, 955. The structure of radialenes is based on oxocarbon and pseudooxocarbon compounds. Oxocarbon- and pseudooxocarbon compounds are sufficiently known as non-benzoid aromatics, see, e.g., G. Seitz, Nachr. Chem. Tech. Lab. 28 (1980), pages 804-807. The first oxocarbon compound, potassium croconate, was produced by L. Gmelin in 1825 from potash and coal. Those compounds, in which at least one oxygen atom is replaced by another heteroatom, are designated as pseudooxocarbons, as is readily known to an expert in the art.

It has been known for several years that organic semiconductors can be heavily influenced regarding their electrical conductivity by doping. Such organic semiconductive matrix materials can be built up either from compounds with good electron donor properties or from compounds with good electron acceptor properties. Strong electron acceptors such as tetracyanoquinonedimethane (TCNQ) or 2,3,5,6-tetrafluoro-tetracyano-1,4-benzoquinonedimethane (F4TCNQ) have become known for the doping of electron donor materials (HT), M. Pfeiffer, A. Beyer, T. Fritz, K. Leo, Appl. Phys. Lett., 73 (22), 3202-3204 (1998). and J. Blochwitz, M. Pfeiffer, T. Fritz, K. Leo, Appl. Phys. Lett., 73 (6), 729-731 (1998). The acceptor molecules generate so-called holes by electron transfer processes in electron donor-like base materials (hole transport materials) and the conductivity of the base material is more or less significantly changed depend on the number and mobility of the holes. For example, N,N'-perarylated benzidines such as TPD or N,N',N"-perarylated starburst compounds such as the substance TDATA, or, however, also certain metal phthalocyanines, such as in particular zinc phthalocyanine ZnPc are known as matrix material with hole transport properties.

However, the previously described compounds have disadvantages for a technical use in the production of doped semiconductive organic layers or of corresponding electronic components with such doped layers since the manufacturing processes in large-scale production plants or those on a technical scale can not always be sufficiently precise, which results in high control- and regulating expense within the processes for achieving a desired product quality or in undesired tolerances of the products. Furthermore, there are disadvantages in the use of previously known organic acceptors with regard to electronic components such as light-emitting diodes (OLEDs), field effect transistors (FET) or solar cells themselves since the cited production difficulties in the handling of the doping agents can lead to undesired irregularities in the electronic components or in undesired ageing effects of the electronic components. However, it should be considered at the same time that the doping agents to be used have extremely high electron affinities (reduction potentials) and other properties suitable for the application case since, e.g., the doping agents also co-determine the conductivity or other electrical properties of the organic semiconductive layer under given conditions. The energetic positions of the HOMO (highest occupied molecular orbital) of the matrix material and of the LUMO (lowest unoccupied molecular orbital) of the doping agent are decisive for the doping effect.

The present invention has the task of overcoming the disadvantages of the state of the art, in particular to make new organic mesomeric compounds available that can be used in particular as doping agent for the doping of organic semiconductors, that can furthermore be more readily handled in the production process and that result in electronic components whose organic semiconductive materials can be reproducibly manufactured. Especially, hole transport materials with a deep HOMO shall be dopable by the new organic mesomeric compounds.

This task is solved by the independent claims of the present application. Preferred embodiments are disclosed in the subclaims.

In the compounds in accordance with the invention the position of the LUMO is so low that further technically interesting hole transport materials can now be efficiently doped for the first time. Due to the extremely low position of the LUMO and to the associated high reduction potential of the compounds even performance efficiencies of solar cells can be significantly improved. The doping effect of a certain magnitude (e.g. a doped layer of a certain conductivity) can be achieved with a substantially lower amount of dopant material to be used compared to conventional dopant under otherwise unchanged conditions. In addition, these compounds are extremely diffusion-stable in organic layers on account of their high polarity. By making available radialenes as doping agents, these make possible a sufficient electrical conductivity of the organic semiconductive matrix given advantageous electron affinity of the doping agents in the particular components at low diffusion coefficients that ensure a component structure that is stable in time. Furthermore, the charge carrier injection of contacts into the doped layer can be improved by the doping agents. Furthermore, the doped organic semiconductive material and the resulting electronic component can have an improved long-time stability on account of the compounds used in accordance with the invention. This concerns, e.g., a reduction or loss of the conductivity over time. This furthermore concerns the stability of the doped layer that is arranged adjacent to non-doped layers of an electro-optical component so that electro-optical components with increased long-time stability of the electro-optical properties such as light emission quantum yield or, effectiveness of a solar cell or the like result.

[3]-radialene compounds[3]-radialene compounds as disclosed in the claims as disclosed in the claims have been found to be specifically useful to perform the invention.Those compounds are able to dope all common OLED hole transport materials. Especially, hole transport materials with a deep HOMO can be doped.

[3]-radialene compounds as disclosed in the claims are strong electron acceptors and form easily radical ion salts (where the [3]-radialene compound can carry for instance one, two or more negative charges) or charge-transfer complexes with electron donor compounds. Such radical ion salts or charge-transfer complexes have a variety of different useful applications such as to for charge injection layers, charge transport layers, organic conductor bodies, ferromagnetic bodies, or electrochromic or photochromic bodies.

By performing doping experiments, it was found that the compounds used in the examples below give good doping properties. It is especially important to note that the substitution pattern defined by those compounds give strongly accepting compounds with a reduction potential in the range of 0V vs. Fc/Fc+ to 0.4V vs. Fc/Fc+. Fc/Fc+ denoted as usual the Ferrocene/ Ferrocenium redox couple. Reduction potentials can be considered as measures for the LUMO of a molecule. Favorable substitution patterns involve six-membered (hetero-) cycles fully substituted with acceptor units such as cyano, fluoro, chloro, bromo and the like, as functional unit in the cyclopropane compound. The six-membered (hetero-) cycles can be for instance perfluoropyridin-4-yl, tetrafluoro-4-(trifluoromethyl)phenyl), 4-cyanoperfluorophenyl, dichloro-3,5-difluoro-4-(trifluoromethyl)phenyl, and perfluorophenyl.

For p-doped OLED or organic solar cell, often hole-injecting materials such as phthalocyanine copper complex (CuPc), 4,4',4"-tris(N-3-methylphenyl-N-phenyl-amino)triphenylamine (m-MTDATA), 4,4',4"-tris(N-(2-naphthyl)-N-phenyl-amino)triphenylamine (2-TNATA) or MeO-TPD (N,N,N',N'-tetrakis(4-methoxy-phenyl)benzidine), or Spiro-TTB (2,2',7,7'-Tetrakis-(N,N-diphenylamino)-9,9'-spirobifluoren, also called Spiro-TTP) are doped by acceptor materials. The layer sequence is then for instance: Anode/ p-doped HIL/ EBL/ EML/ ETL/ LiF/ Cathode. Herein, HIL denotes a hole injection layer, EBL denotes a electron blocking layer, EML denotes a (light) emitting layer, ETL denoted an electron transport layer, LiF denotes Lithium fluoride layer. Such HIL materials have typically a relatively low oxidation potential in the range of 0V to 0.1V vs. Fc/Fc+. Oxidation potential can be considered as a measure for the HOMO of a molecule. There is a need, however, to achieve good doping results also in host materials which are conventionally used as HTL or EBL materials. They often have a higher oxidation potential in the range of 0.2 to 0.4 V vs. Fc/Fc+. It is remarkable, that the selected dopants provide the same high conductivities in a HIL type host and a HTL type host. HTL type materials are for instance: N,N'-Bis(naphthalen-1-yl)-N,N'-bis(phenyl)-benzidine, N,N'-Bis(naphthalen-1-yl)- N,N'-bis(phenyl)-9,9-spiro-bifluorene, 9,9-Bis[4-(N,N-bis-biphenyl-4-yl-amino)phenyl]-9H-fluorene, N,N'-bis(phenanthren-9-yl)-N,N'-bis(phenyl)-benzidine, 2,2'-Bis[N,N-bis(biphenyl-4-yl)amino]9,9-spiro-bifluorene, 1,3,5-tris{4-[bis(9,9-dimethyl-fluoren-2-yl)amino]phenyl}benzene, tri(terphenyl-4-yl)amine.

The deposition rate on a substrate with the compound used in accordance with the invention can be determined, e.g., using a quartz thickness monitor, as is customarily used, e.g., in the production of OLEDs. In particular, the ratio of the deposition rates of matrix materials and doping agent can be measured by independent measurements of them using two separate quartz thickness monitors in order to adjust the doping ratio.

It is understood that the compounds used in accordance with the invention are preferably such that they evaporate more or less or practically non-decomposed. However, if necessary, even purposeful precursors can be used as doping source that release the compounds used in accordance with the invention, e.g., acid addition salts, e.g., of a volatile or non-volatile inorganic or organic acid, or their charge transfer complexes, which acids and/or electron donors are preferably not volatile or only slightly volatile or the charge transfer complex itself acts as doping agent.

The doping agent is preferably selected in such a manner that it generates a conductivity just as high as or preferably higher than F4TCNQ under conditions that are otherwise the same such as, in particular, doping concentration (molar ratio, doping agent:matrix, layer thickness, current strength) at a given matrix material (e.g., zinc phthalocyanine or another matrix material cited further below), e.g., a conductivity (s/cm) greater than/equal to 1.1 times, 1.2 times or greater than/equal to 1.5 times or twice that of F4TCNQ as doping agent.

The doping agent used in accordance with the invention is preferably selected in such a manncr that the semiconductive organic material doped with it still has ≥20%, preferably ≥30%, especially preferably ≥50% or 60% of the conductivity (s/cm) of the value at 100°C after a temperature change of 100°C to RT (20°C).

### Preparation of oxocarbon-, pseudooxocarbon- and radialene structures

The first oxocarbon compound, potassium croconate, was produced by L. Gmelin in 1825 from potash and coal. Oxocarbons and their esters and halogenides preferably react with electron-rich compounds such as aliphatic and aromatic amines, aromatics and heteroaromatics. A.H. Schmidt, Synthesis (1980) 961. The reaction products from tetrachlorocyclopropene and phenols in the presence of Lewis acids or CH-acidic compounds by strong bases, such as, e.g., arylacetonitriles, 1,3-diketones, cyclopentadienes, malonodinitriles, acceptor-substituted diarylmethanes, electron-poor diheteroarylmethanes are especially suitable for applications in accordance with the invention. [3]-Radialenes are obtained after oxidation has taken place, R.West et al. J. Org. Chem. (1975) 40 2295; T.Kazuka, T.Shinji J. Chem. Soc. Chem. Commun.(1994) 519; T.Fukunaga et al. JACS (1976) 98 610.

### Matrix materials

The present invention describes suitable doping agents for organic semiconductive materials such as hole transport materials **HT** that are customarily used in OLEDs or organic solar cells. The semiconductive materials are preferably intrinsically hole-conducting. The following gives an exemplary description of materials that can be applied in conjunction with doping agents of the radialene type in accordance with the invention.

The matrix material can consist partially (> 10 or > 25% by weight) or substantially (> 50 % by weight or > 75% by weight) or totally of a metal phthalocyanine complex, a porphyrine complex, especially metal porphyrine complex, oligothiophene-, oligophenyl-, oligophenylene vinylene- or oligofluorene compound, in which the oligomer preferably comprises 2-500 or more, preferably 2-100 or 2-50 or 2-10 or more monomeric units. The oligomer can also comprise > 4, > 6 or > 10 or more monomeric units, in particular also for the above-indicated ranges, thus, e.g., 4 or 6-10 monomeric units, 6 or 10-100 monomeric units or 10-500 monomeric units. Polymeric matrix materials can also be used. The monomers and oligomers can be substituted or unsubstituted and even block- or mixed polymerizates of the cited oligomers can be present as well as a compound with a triarylamine unit or a spiro-bifluorene compound. The cited matrix materials can also be present in combination with each other, optionally also in combination with other matrix materials. The matrix materials can have electron-donating substitutents such as alkyl- or alkoxy groups that have a reduced ionizing energy or reduce the ionizing energy of the matrix material.

The metal phthalocyanine complexes or porphyrine complexes used as matrix material can have a main group metal atom or subgroup metal atom. The metal atom Me can be coordinated 4-, 5- or 6-fold, e.g., in the form of oxo- (Me=O), dioxo- (O=Me=O) imine-, diimine-, hydroxo-, dihydroxo-, amino- or diamino complexes, without being limited to them. The phthalocyanine complex or porphyrine complex can each be partially hydrogenated, however, the mesomeric ring system is preferably not disturbed. The phthalocyanine can contain, e.g., magnesium, zinc, iron, nickel, cobalt, magnesium, copper or vanadyl (=VO) as central atom. The same or other metal atoms or oxometal atoms can be present in the case of porphyrine complexes.

In particular, such dopable hole transport materials **HT** can be arylated benzidines, e.g., N,N'-perarylated benzidines or other diamines such as of the type **TPD** (in which one, several or all of the aryl groups can have aromatic heteroatoms), suitable arylated starburst compounds such as N,N',N"-perarylated starburst compounds such as the compound **TDATA** (in which one, several or all of the aryl groups can have aromatic heteroatoms). The aryl groups can comprise phenyl, naphthyl, pyridine, quinoline, isoquinoline, peridazine, pyrimidine, pyrazine, pyrazole, imidazole, oxazole, furan, pyrrole, indole or the like, especially for each of the above-cited compounds. The phenyl groups of the particular compounds can be partially or completely replaced by thiophene groups.

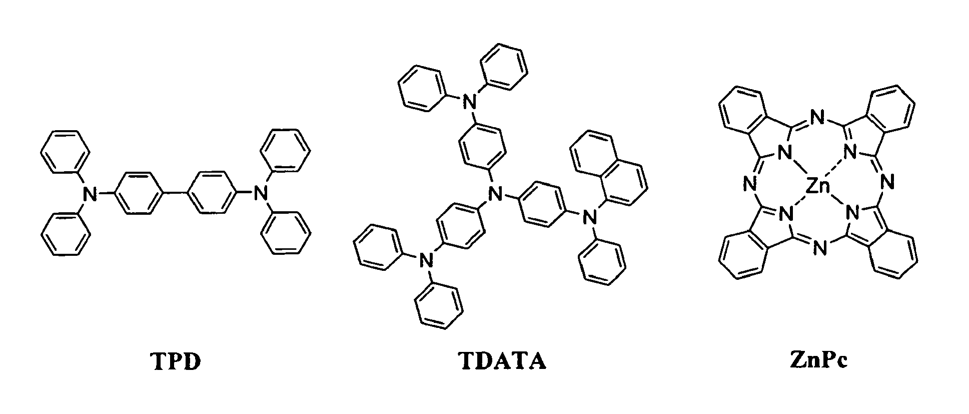

It is understood that even other suitable organic matrix materials, in particular hole-conducting materials can be used that have semiconductive properties.

### Doping

The doping can take place in particular in such a manner that the molar ratio of matrix molecule to doping agent, or in the case of oligomeric matrix materials the ratio of matrix monomer number to doping agent is 1:100000, preferably 1:10000, especially preferably 1:5 to 1:1000, e.g., 1:10 to 1:100, e.g., ca. 1:50 to 1:100 or also 1:25 to 1:50.

### Evaporation of the doping agents

The doping of the particular matrix material (preferably indicated here as hole-conducting matrix material HT) with the doping agents to be used in accordance with the invention can be produced by one or a combination of the following processes:
a) Mixed evaporation in the vacuum with a source for HT and one for the doping agent.
b) Sequential deposition of HT and doping agent with subsequent inward diffusion of the doping agent by thermal treatment
c) Doping of an HT layer by a solution of doping agent with subsequent evaporation of the solvent by thermal treatment
d) Surface doping of an HT layer by a layer of doping agent applied on either or both surfaces of the HT layer.
e) Making a solution of host and dopant and form a film from the solution for instance by coating, casting or printing techniques or other film making techniques known to a person skilled in the art.

The doping can take place in such a manner that the doping agent is evaporated out of a precursor compound that releases the doping agent under heating and/or irradiation. The irradiation can take place by electromagnetic radiation, especially visible light, UV light or IR light, e.g., by laser light or also by other radiation types. The heat necessary for evaporation can substantially be made available by the irradiation and can also be radiated in a purposeful manner into certain bands of the compounds or precursors or compound complexes such as charge transfer complexes to be evaporated in order to facilitate the evaporation of the compounds by dissociation of the complexes by conversion into excited states. It is understood that the evaporation conditions described in the following are directed to those without irradiation and that uniform evaporation conditions are to be used for purposes of comparison.

For example, the following can be used as precursor compounds:
a) Mixtures or stoichiometric or mixed crystalline compounds of the doping agent and an inert, non-volatile substance, e.g., a polymer, molecular sieve, aluminum oxide, silica gel, and oligomers or another organic or inorganic substance with high evaporation temperature, in which the doping agent is bound primarily by van der Waals forces and/or hydrogen bridge bonding to this substance.
b) Mixture or stoichiometric or mixed crystalline compound of the doping agent and one non-volatile compound V more or less of the electron donor type, in which a more or less complete charge transfer occurs between the doping agent and the compound V as in charge transfer complexes with more or less electron-rich polyaromatics or heteroaromatics or another organic or inorganic substance with high evaporation temperature.
c) Mixture or stoichiometric or mixed crystalline compound of the doping agent and a substance that evaporates together with the doping agent and has the same or higher ionizing energy as the substance HT to be doped, so that the substance does not form a trap for holes in the organic matrix material. According to the invention the substance can also be identical to the matrix material here, e.g., be a metal phthalocyanine or benzidine derivative. Further suitable volatile co-substances such as hydroquinones, 1,4-phenylene diamines or 1-amino-4-hydroxybenzene or other compounds form quinhydrones or other charge transfer complexes.

### Electronic component

A plurality of electronic components or equipment containing them can be produced using the organic compounds in accordance with the invention for producing doped organic semiconductive materials that can be arranged in particular in the form of layers or electrical line paths. In particular, the doping agents in accordance with the invention can be used to produce organic, light-emitting diodes (OLED), organic solar cells, organic diodes, especially those with a high rectification ratio such as 10³-10⁷, preferably 10⁴-10⁷ or 10⁵-10⁷ or organic field effect transistors. The conductivity of the doped layers and/or the improvement of the charge carrier injection of contacts into the doped layer can be improved by the doping agents in accordance with the invention. In particular in the case of OLEDs or solar cells the component can have a pin structure (the device has a one or more p-doped hole transport layers and/or one or more n-doped electron transport layers) or an inverted structure (the top-electrode and hole transport layer are located on the same side from the light emitting or light harvesting layer while the substrate is on the opposite side) without being limited to them. An injection layer can be made, for instance, by forming a layer containing or consisting of the organic compounds in accordance with the invention between an electrode and a charge transporting layer. However, the use of the doping agents in accordance with the invention is not limited to the advantageous exemplary embodiments cited above.

### Exemplary embodiments

The invention will be explained in detail with a few exemplary embodiments.

The compounds in accordance with the invention will now be used in the following manner as doping agents for different hole conductors that for their part are used for constructing certain microelectronic or optoelectronic components such as, e.g., an OLED. The doping agents can be co-evaporated with the hole transport materials of the matrix in high vacuum (ca. 2 x 10⁻⁴ Pa) by thermal evaporation. A typical deposition rate for the matrix material is 0.2 nm/s (density ca. 1.5 g/cm³). The evaporation rates for the doping agents can vary between 0.001 and 0.5 nm/s (assuming the same density) in accordance with the desired doping ratio.

In the following examples the current measurements were carried out over a current path of the doped HT material 1 mm long and ca. 0.5 mm wide at 1V. under these conditions ZnPc conducts practically no electrical current.

### Synthesis of examples:

### 1. Ethyl 2-cyano-2-aryl acetates a), b), c), d) and e)

### General procedure:

To a solution of 207 mmol of the either starting material A, B, C, D or E and 250 mmol of potassium carbonate in 370 ml of dimethylformamide 207 mmol of cyano acetic ester in 50 ml of dimethylformamid were added quickly. The mixture was allowed to stirr for 48 h at room temperature. Then the reaction suspension was poured into a 3L beaker with 1L of ice water. While stirring the solution was acidified with 100mL of conc. acetic acid. The aqueous solution was extracted four times with chloroform in this order (250mL, 150mL, 100mL, 100mL). After drying the combined organic layers with magnesium sulphate the solvent was removed in vacuum. The remaining oil was used in the next step without any further purification.

### a) Ethyl 2-cyano-2-(perfluorophenyl) acetate:

Hexafluorobenzene (A) has been used as starting material. 51.9 g of the ester were obtained according to the procedure described above.

### b) Ethyl 2-cyano-2-(perfluoropyridin-4-yl) acetate:

Pentafluoropyridin (B) has been used as starting material. 47.7 g of the ester were obtained according to the procedure described above.

### c) Ethyl 2-cyano-2-(4-cyanoperfluorophenyl) acetate:

Pentafluorobenzonitril (C) has been used as starting material. 54.3 g of the ester were obtained according to the procedure described above.

### d) Ethyl 2-cyano-2-(4-trifluoromethylperfluorophenyl) acetate:

Octafluorotoluol (D) has been used as starting material. 66.8 g of the ester were obtained according to the procedure described above.

### e) Ethyl 2-cyano-2-(4-trifluoromethyl-2,6-dichloro-3,5-difluorophenyl) acetate:

4-trifluoromethyl-2,6-dichloro-1,3,5-trifluorobenzene (E) has been used as starting material. 64.8 g of the ester were obtained according to the procedure described above.

### 2. Aryl acetonitriles f), g), h), i) and k):

### General procedure:

In a 250mL round bottom flask the whole amount of the ethyl 2-cyano-2-aryl acetate a), b), c) d) or e) as synthesized above was dissolved in 84 ml of acetic acid (50%) together with 4.15 ml of sulfuric acid (conc.). The mixture was heated on reflux for 16 hours. After cooling to room temperature the mixture was poured into a 500mL beaker with 120mL of ice water and stirred over a period of 30 min. The organic layer was separated and the aqueous layer extracted with 100mL of chloroform. The combined organic layers were washed with 100mL of water and with 100mL of saturated sodium bicarbonate solution. After drying the organic layer with magnesium sulphate the solvent was removed in vacuum to give brown coloured oil. Distillation in vacuum gave a colourless slow solidifying liquid.

### f) Pentafluorophenyl acetonitrile:

Ethy-2-cyano-2-(perfluorophenyl) acetate (a) was used as starting material. 36.4 g (176 mmol; 85% based on starting material A) of the aryl acetontrile were obtained according to the procedure described above.

### g) 4-(cyanomethyl)-2,3,5,6-tetrafluoropyridine:

Ethyl 2-cyano-2-(perfluoropyridin-4-yl) acetate (b) was used as starting material. 33.1 g (174 mmol; 84% based on starting material B) of the aryl acetontrile were obtained according to the procedure described above.

### h) 4-(cyanomethyl)-2,3,5,6-tetrafluorobenzonitril:

Ethyl-2-cyano-2-(4-cyanoperfluorophenyl) acetate (c) was used as starting material. 39.0 g (182 mmol; 88% based on starting material C) of the aryl acetontrile were obtained according to the procedure described above.

### i) 2-(2,3,5,6-tetrafluoro-4-(trifluoromethyl)phenyl)acetonitrile:

Ethyl-2-cyano-2-(4-trifluoromethylperfluorophenyl) acetate (d) was used as starting material. 48.8 g (190 mmol; 92% based on starting material D) of the aryl acetontrile were obtained according to the procedure described above.

### k) (4-trifluoromethyl-2,6-dichloro-3,5-difluorophenyl) acetonitrile:

Ethyl-2-cyano-2-(4-trifluoromethyl-2,6-dichloro-3,5-difluorophenyl) acetate (e) was used as starting material. 53.4 g (184 mmol; 89% based on starting material E) of the aryl acetontrile were obtained according to the procedure described above.

### 3. [3]-radialenes l), m), n), o) and p)

### General procedure:

Lithium hydride (98%) is suspended in 600mL of 1,2- dimethoxyethane and cooled to 0°C. 152 mmol of aryl acetonitrile f), g), h), i) or k) was dissolved in 60mL of 1,2-dimethoxyethane and added over a period of 10 to 15 min. The ice bath has been removed and the reaction was allowed to warm up over 45 min. After 15 min stirring at room temperature the mixture was cooled to 0°C again. 7.12 g (40.0 mmol) of perchlorocycloprop-1-ene in 40mL of 1,2-dimethoxyethane were added dropwise. The colour of the solution turned to dark red. The dark solution was kept on stirring for 44 h while warming up to room temperature. Then the reaction suspension was poured into a 2 L beaker with 1.2 L of ice water. The stirring solution was acidified with concentrated hydrochloric acid to pH=1 (240 mL HCl) and extracted with ethyl acetate (3x500 mL). The combined organic layers were washed in the following order with brine, water and bicarbonate solution and then with water again. The combined organic layers were dried with magnesium sulphate and the solvent was carefully removed in vacuum to give a dark coloured material which was directly used in the next transformation without any further purification.

The dark coloured material was dissolved in 1400mL of acetic acid (100%) and treated with a mixture of 360mL hydrobromic acid (48%) and 120mL of nitric acid (65%) prepared approximately ten minutes before. The resulting mixture was stirred for 1.5 hours. The mixture was filtrated and the resulting orange precipitate was washed with water and dried in vacuum to afford the crude material. The crude material was purified by gradient sublimation.

### 1) (2E,2'E,2"E)-2,2',2"-(cyclopropane-1,2,3-triylidene)tris(2-(perfluorophenyl)-acetonitrile)

Pentafluorophenyl acetonitrile (f) was used as starting material. 9.37 g (14.4 mmol; 36% based on tetrachlorocyclopropene) of the [3]radialene were obtained after gradient sublimation according to the procedure described above.
Mp. 211°C

### m) (2E,2'E,2"E)-2,2',2"-(cyclopropane-1,2,3-triylidene)tris(2-(perfluoropyridin-4-yl)-acetonitrile)

4-(cyanomethyl)-2,3,5,6-tetrafluoropyridine (g) was used as starting material. 8.40 g (14.0 mmol; 35% based on tetrachlorocyclopropene) of the [3]radialene were obtained after gradient sublimation according to the procedure described above.
Mp. 140°C

### n) (2E,2'E,2"E)-2,2',2"-(cyclopropane-1,2,3-triylidene)tris(2-(4-cyanoperfluorophenyl)-acetonitrile)

4-(cyanomethyl)-2,3,5,6-tetrafluorobenzonitrile (h) was used as starting material. 10.2 g (15.2 mmol; 38% based on tetrachlorocyclopropene) of the [3]radialene were obtained after gradient sublimation according to the procedure described above.
Mp. 316°C

### o) (2E,2'E,2"E)-2,2',2"-(cyclopropane-1,2,3-triylidene)tris(2-(2,3,5,6-tetrafluoro-4-(trifluoromethyl)phenyl)-acetonitrile)

2-(2,3,5,6-tetrafluoro-4-(trifluoromethyl)phenyl)acetonitrile (i) was used as starting material. 12.8 g (16.0 mmol; 40% based on tetrachlorocyclopropene) of the [3]radialene were obtained after gradient sublimation according to the procedure described above.
Mp. 197°C

### p) (2E,2'E,2"E)-2,2',2"-(cyclopropane-1,2,3-triylidene)tris(2-(2,6-dichloro-3,5-difluoro-4-(trifluoromethyl)phenyl)-acetonitrile)

(4-trifluoromethyl-2,6-dichloro-3,5-difluorophenyl) acetonitrile (k) was used as starting material. 7.92 g (8.80 mmol; 22% based on tetrachlorocyclopropene) of the [3]radialene were obtained after gradient sublimation according to the procedure described above.
Mp. 220 °C

### Doping Examples:

### Example 1:

A mixed layer of Spiro-TTB as a host material and (2E,2'E,2"E)-2,2',2"-(cyclopropane-1,2,3-triylidene)tris(2-(perfluorophenyl)-acetonitrile) (I) as a dopant material have been made by mixed thermal evaporation on a glass substrate in a high vacuum chamber. The doping concentration was 5mol%, the film thickness 50nm. The glass substrate has two ITO stripes with a distance of 1mm as electrodes for the film. From the current-voltage characteristics of the film and the geometry of the sample, the conductivity of the mixed layer was determined to be 1.7·10⁻⁶ S/cm.

### Example 2:

Another film has been made like in example 1. As the dopant, (2E,2'E,2"E)-2,2',2"-(cyclopropane-1,2,3-triylidene)tris(2-(perfluoropyridin-4-yl)-acetonitrile) (m) was used. The conductivity of the film was 4.3·10⁻⁵ S/cm.

### Example 3:

Another film has been made like in example 1. As the dopant, (2E,2'E,2"E)-2,2',2"-(cyclopropane-1,2,3-triylidene)tris(2-(perfluoropyridin-4-yl)-acetonitrile) (m) was used. As the host material, N,N'-Bis(naphthalen-1-yl)-N,N'-bis(phenyl)-benzidine was used. The conductivity of the film was 1.3·10⁻⁵ S/cm.

### Example 4:

Another film has been made like in example 3. As the dopant, (2E,2'E,2"E)-2,2',2"-(cyclopropane-1,2,3-triylidene)tris(2-(4-cyanoperfluorophenyl)-acetonitrile) (n) was used. The doping concentration was 10mol%. The conductivity of the film was 6.8·10⁻⁵ S/cm.

### Example 5:

Another film has been made like in example 3. As the dopant, (2E,2'E,2"E)-2,2',2"-(cyclopropane-1,2,3-triylidene)tris(2-(2,3,5,6-tetrafluoro-4-(trifluoromethyl)phenyl)-acetonitrile) (o) was used. The doping concentration was 10mol%. The conductivity of the film was 4·10⁻⁵ S/cm.

### Example 8:

Another film has been made like in example 3. As the dopant, (2E,2'E,2"E)-2,2',2"-(cyclopropane-1,2,3-triylidene)tris(2-(2,6-dichloro-3,5-difluoro-4-(trifluoromethyl)phenyl)-acetonitrile) (p) was used. The doping concentration was 10mol%. The conductivity of the film was 1.3·10⁻⁵ S/cm.

### Example 9:

Another film has been made like in example 8. As the host, 9,9-Bis[4-(N,N-bis-biphenyl-4-yl-amino)phenyl]-9H-fluorene was used. The conductivity of the film was 1.2·10⁻⁵ S/cm.

The features of the invention disclosed in the previous description and in the claims can be essential individually as well as in any combination for the realization of the invention in its various embodiments.

## Claims

1. Use of an organic mesomeric compound as organic doping agent for the doping of an organic semiconductive matrix material, as blocker layer, as charge injection layer or as organic semiconductor itself, **characterized in that** the mesomeric compound is a radialene compound with the following formula: 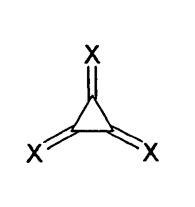 in which each X is 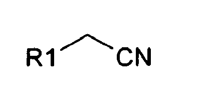 wherein each R₁ being independently selected from aryl and heteroaryl, aryl and heteroaryl being at least partially, preferably completely, substituted with electron acceptor groups.

2. Use according to Claim 1, **characterized in that** the electron acceptor groups are selected from cyano, fluoro, trifluoromethyl, chloro and bromo.

3. Use according to Claim 1 or 2, **characterized in that** R₁ is selected from perfluoropyridin-4-yl, tetrafluoro-4-(trifluoromethyl)phenyl), 4-cyanoperfluorophenyl, dichloro-3,5-difluoro-4-(trifluoromethyl)phenyl, and perfluorophenyl.

4. Radialene compound with the following formula 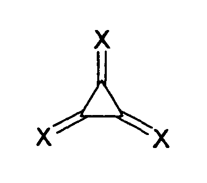 in which each X is 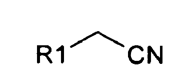 wherein each R₁ being independently selected from aryl and heteroaryl, aryl and heteroaryl being at least partially, preferably completely, substituted with electron acceptor groups.

5. Radialene compound according to claim 4 selected from (2E,2'E,2"E)-2,2',2"-(cyclopropane-1,2,3-triylidene)tris(2-(perfluorophenyl)-acetonitrile), (2E,2'E,2"E)-2,2',2"-(cyclopropane-1,2,3-triylidene)tris(2-(perfluoropyridin-4-yl)-acetonitrile), (2E,2'E,2"E)-2,2',2"-(cyclopropane-1,2,3-triylidene)tris(2-(4-cyanoperfluorophenyl)-acetonitrile), (2E,2'E,2"E)-2,2',2"-(cyclopropane-1,2,3-triylidene)tris(2-(2,3,5,6-tetrafluoro-4-(trifluoromethyl)phenyl)-acetonitrile), and (2E,2'E,2"E)-2,2',2"-(cyclopropane-1,2,3-triylidene)tris(2-(2,6-dichloro-3,5-difluoro-4-(trifluoromethyl)phenyl)-acetonitrile).

6. Use of radialene compounds according to Claim 4 or their radical anionic salts, dianionic salts or their charge transfer complexes with donors as organic conductors, ferromagnets, or electro- or photochromic material.

7. Organic semiconductive material containing at least one organic matrix compound and one doping agent, **characterized in that** the doping agent is one or more radialene compounds according to claim 4.

8. Organic semiconductive material according to Claim 7, **characterized in that** the matrix compound is selected from phthalocyanine copper complex (CuPc), 4,4',4"-tris(N-3-methylphenyl-N-phenyl-amino)triphenylamine (m-MTDATA), 4,4',4"-tris(N-(2-naphthyl)-N-phenyl-amino)triphenylamine (2-TNATA), MeO-TPD (N,N,N',N'-tetrakis(4-methoxy-phenyl)benzidine), (2,2',7,7'-tetrakis-(N,N-diphenylamino)-9,9'-spirobifluoren (spiro-TTB), N,N'-bis(naphthalen-1-yl)-N,N'-bis(phenyl)-benzidine, N,N'-bis(naphthalen-1-yl)-N,N'-bis(phenyl)-9,9-spiro-bifluorene, 9,9-bis[4-(N,N-bis-biphenyl-4-yl-amino)phenyl]-9H-fluorene, N,N'-bis(phenanthren-9-yl)-N,N'-bis(phenyl)-benzidine, 2,2'-bis[N,N-bis(biphenyl-4-yl)amino]9,9-spiro-bifluorene, 1,3,5-tris{4-[bis(9,9-dimethyl-fluorene-2-yl)amino]phenyl}benzene, and tri(terphenyl-4-yl)amine

9. Organic semiconductive material according to Claim 7 or 8, **characterized in that** the molar doping ratio of doping agent to matrix molecule and/or the doping ratio of doping agent to monomeric units of a polymeric matrix molecule is between 1:1 and 1:100,000.

10. Electronic component with an electronically functionally active area, **characterized in that** the electronically functionally active area is produced using at least one or more radialene compounds according to claim 4.

11. Electronic component according to Claim 10, **characterized in that** the electronically functionally active area comprises an organic semiconductive matrix material that is doped with at least one doping agent for changing the electronic properties of the semiconductive matrix material using at least one or more of the radialene compounds according to claim 4.

12. Electronic component according to Claim 10 or 11 in the form of an organic light-emitting diode, a photovoltaic cell, an organic solar cell, an organic diode or an organic field effect transistor.

## Patentansprüche

1. Verwendung einer organischen mesomeren Verbindung als organischer Dotand für das Dotieren eines organischen halbleitenden Matrixmaterials, als Blockerschicht, als Ladungsinjektionsschicht oder als organischer Halbleiter selbst, **dadurch gekennzeichnet, dass** die mesomere Verbindung eine Radialenverbindung mit der folgenden Formel: 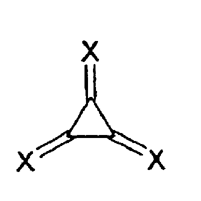 ist, wobei jedes X 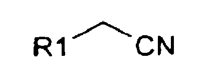 ist, wobei jedes R₁ unabhängig ausgewählt wird aus Aryl und Heteroaryl, wobei Aryl und Heteroaryl wenigstens teilweise, bevorzugt vollständig, mit Elektronenakzeptorgruppen substituiert sind.

2. Verwendung nach Anspruch 1, **dadurch gekennzeichnet, dass** die Elektronenakzeptorgruppen ausgewählt sind aus Cyano, Fluor, Trifluormethyl, Chlor und Brom.

3. Verwendung nach Anspruch 1 oder 2, **dadurch gekennzeichnet, dass** R₁ ausgewählt wird aus Perfluorpyridin-4-yl, Tetrafluor-4-(trifluormethyl)phenyl), 4-Cyanoperfluorphenyl, Dichlor-3,5-difluor-4-(trifluormethyl)phenyl und Perfluorphenyl.

4. Radialenverbindung mit der folgenden Formel 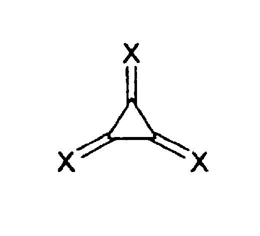 in welcher jedes X 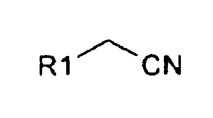 ist, wobei jedes R₁ unabhängig ausgewählt ist aus Aryl und Heteroaryl, wobei Aryl und Heteroaryl wenigstens teilweise, bevorzugt vollständig, mit Elektronenakzeptorgruppen substituiert sind.

5. Radialenverbindung nach Anspruch 4 ausgewählt aus (2E,2'E,2"E)-2,2',2"-(Cyclopropan-1,2,3-triyliden)tris(2-(perfluorphenyl)-acetonitril), (2E,2'E,2"E)-2,2',2"-(Cyclopropan-1,2,3-triyliden)tris(2-(perfluorpyridin-4-yl)-acetonitril), (2E,2'E,2"E)-2,2',2"-(Cyclopropan-1,2,3-triyliden)tris(2-(4-cyanoperfluorphenyl)-acetonitril), (2E,2'E,2"E)-2,2',2"-(Cyclopropan-1,2,3-triyliden)tris(2-(2,3,5,6-tetrafluor-4-(trifluormethyl)phenyl)-acetonitril) und (2E,2'E,2"E)-2,2',2"-(Cyclopropan-1,2,3-triyliden)tris(2-(2,6-dichlor-3,5-difluor-4-(trifluormethyl)phenyl)-acetonitril).

6. Verwendung von Radialenverbindungen nach Anspruch 4 oder deren radikalischen anionischen Salze, dianionischen Salze oder deren Ladungstransferkomplexen mit Donoren als organische Leiter, Ferromagneten oder elektro- photochromes Material.

7. Organisches halbleitendes Material enthaltend wenigstens eine organische Matrixverbindung und einen Dotanden, **dadurch gekennzeichnet, dass** der Dotand eine oder mehrere Radialenverbindungen nach Anspruch 4 ist.

8. Organisches halbleitendes Material nach Anspruch 7, **dadurch gekennzeichnet, dass** die Matrixverbindung ausgewählt ist aus Phthalocyaninkupferkomplex (CuPc), 4,4',4"-Tris(N-3-methylphenyl-N-phenyl-amino)triphenylamin (m-MTDATA), 4,4',4"-Tris(N-(2-naphthyl)-N-phenyl-amino)triphenylamin (2-TNATA), MeO-TPD (N,N,N',N'-Tetrakis(4-methoxy-phenyl)benzidin), (2,2',7,7'-Tetrakis-(N,N-diphenylamino)-9,9'-spirobifluoren (spiro-TTB), N,N'-Bis(naphthalen-1-yl)-N,N'-bis(phenyl)-benzidin, N,N'-Bis(naphthalen-1-yl)-N,N'-bis(phenyl)-9,9-spiro-bifluoren, 9,9-Bis[4-(N,N-bis-biphenyl-4-yl-amino)phenyl]-9H-fluoren, N,N'-Bis(phenanthren-9-yl)-N,N'-bis(phenyl)-benzidin, 2,2'-Bis[N,N-bis(biphenyl-4-yl)amino]9,9-spiro-bifluoren, 1,3,5-Tris{4-[bis(9,9-dimethyl-fluoren-2-yl)amino]phenyl}benzol, und Tri(terphenyl-4-yl)amin.

9. Organisches halbleitendes Material nach Anspruch 7 oder 8, **dadurch gekennzeichnet, dass** das molare Dotierungsverhältnis von Dotand zu Matrixmolekül und/oder das Dotierungsverhältnis von Dotand zu monomeren Einheiten eines polymeren Matrixmoleküls zwischen 1:1 und 1:100.000 ist.

10. Elektronisches Bauelement mit einem elektronisch funktionell wirksamen Bereich, **dadurch gekennzeichnet, dass** der elektronisch funktionell wirksame Bereich hergestellt ist unter Verwendung wenigstens einer oder mehrerer Radialenverbindungen nach Anspruch 4.

11. Elektronisches Bauelement nach Anspruch 10, **dadurch gekennzeichnet, dass** der elektronisch funktionell wirksame Bereich ein organisches halbleitendes Matrixmaterial umfasst, das mit wenigstens einem Dotanden zum Ändern der elektronischen Eigenschaften des halbleitenden Matrixmaterials unter Verwendung wenigstens einer oder mehrerer der Radialenverbindungen nach Anspruch 4 dotiert ist.

12. Elektronisches Bauelement nach Anspruch 10 oder 11 in der Form einer organischen lichtemittierenden Diode, einer Photovoltaikzelle, einer organischen Solarzelle, einer organischen Diode oder eines organischen Feldeffekttransistors.

## Revendications

1. Utilisation d'un composé mésomère organique en tant qu'agent dopant organique pour le dopage d'un matériau matriciel semi-conducteur organique, en tant que couche de blocage, en tant que couche d'injection de charge ou en tant que semi-conducteur organique lui-même, **caractérisée en ce que** le composé mésomère est un composé de radialène répondant à la formule suivante : 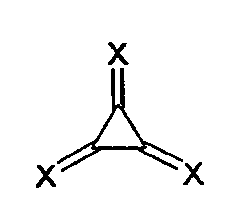 dans laquelle chaque X est 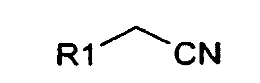 chaque R₁ étant indépendamment choisi parmi un groupe aryle et un groupe hétéroaryle, les groupes aryle et hétéroaryle étant au moins partiellement, de préférence totalement, substitués avec des groupes accepteurs d'électrons.

2. Utilisation selon la revendication 1, **caractérisée en ce que** les groupes accepteurs d'électrons sont choisis parmi les groupes cyano, fluoro, trifluorométhyle, chloro et bromo.

3. Utilisation selon la revendication 1 ou 2, **caractérisée en ce que** R₁ est choisi parmi les groupes perfluoropyridin-4-yl, tétrafluoro-4-(trifluorométhyl)phényle), 4-cyanoperfluorophényle, dichloro-3,5-difluoro-4-(trifluorométhyl)phényle, et perfluorophényle.

4. Composé de radialène répondant à la formule suivante 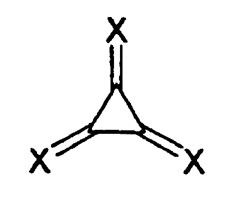 dans laquelle chaque X est 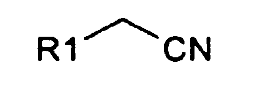 chaque R₁ étant indépendamment choisi parmi un groupe aryle et un groupe hétéroaryle, les groupes aryle et hétéroaryle étant au moins partiellement, de préférence totalement, substitués avec des groupes accepteurs d'électrons.

5. Composé de radialène selon la revendication 4, choisi parmi le (2E,2'E,2"E)-2,2',2"-(cyclopropane-1,2,3-triylidène)tris(2-(perfluorophényl)-acétonitrile), le (2E,2'E,2"E)-2,2',2"-(cyclopropane-1,2,3-triylidène)tris(2-(perfluoropyridin-4-yl)-acétonitrile), le (2E,2'E,2"E)-2,2',2"-(cyclopropane-1,2,3-triylidène)tris(2-(4-cyanoperfluorophényl)-acétonitrile), le (2E,2'E,2"E)-2,2',2"-(cyclopropane-1,2,3-triylidène)tris(2-(2,3,5,6-tétrafluoro-4-(trifluorométhyl)phényl)-acétonitrile), et le (2E,2'E,2"E)-2,2',2"-(cyclopropane-1,2,3-triylidène)tris(2-(2,6-dichloro-3,5-difluoro-4-(trifluorométhyl)phényl)-acétonitrile).

6. Utilisation de composés de radialène selon la revendication 4, ou de leurs sels d'anions radicaux, de leurs sels de dianions ou de leurs complexes de transfert de charges avec des donneurs tels que des conducteurs organiques, des ferro-aimants, ou un matériau électro- ou photochrome.

7. Matériau semi-conducteur organique contenant au moins un composé matriciel organique et un agent dopant, **caractérisé en ce que** l'agent dopant est un composé de radialène ou plus selon la revendication 4.

8. Matériau semi-conducteur organique selon la revendication 7, **caractérisé en ce que** le composé matriciel est choisi parmi un complexe phtalocyanine-cuivre (CuPc), la 4,4',4"-tris(N-3-méthylphényl-N-phényl-amino)triphénylamine (m-MTDATA), la 4,4',4"-tris(N-(2-naphtyl)-N-phényl-amino)triphénylamine (2-TNATA), la MeO-TPD (N,N,N',N'-tétrakis(4-méthoxy-phênyl)benzidine), le (2,2',7,7'-tétrakis-(N,N-diphénylamino)-9,9'-spirobifluorène (spiro-TTB), la N,N'-bis(naphtalen-1-yl)-N,N'-bis(phényl)-benzidine, le N,N'-bis(naphtalen-1-yl)-N,N'-bis(phényl)-9,9-spiro-bifluorène, le 9,9-bis[4-(N,N-bis-biphényl-4-yl-amino)phényl]-9H-fluorène, la N,N'-bis(phénanthren-9-yl)-N,N'-bis(phényl)-benzidine, le 2,2'-bis [N,N-bis(biphényl-4-yl)amino]9,9-spiro-bifluorène, le 1,3,5-tris {4-[bis(9,9-diméthyl-fluorène-2-yl)amino]phényl}benzène, et la tri(terphényl-4-yl)amine.

9. Matériau semi-conducteur organique selon la revendication 7 ou 8, **caractérisé en ce que** le rapport molaire de dopage de l'agent dopant sur la molécule matricielle et/ou le rapport de dopage de l'agent dopant sur les unités monomères d'une molécule matricielle polymère est compris entre 1/1 et 1/100 000.

10. Composant électrique ayant une zone électriquement et fonctionnellement active, **caractérisé en ce que** la zone électriquement et fonctionnellement active est produite en utilisant un composé de radialène ou plus selon la revendication 4.

11. Composant électronique selon la revendication 10, **caractérisé en ce que** la zone électriquement et fonctionnellement active comprend un matériau matriciel semi-conducteur organique qui est dopé avec au moins un agent dopant pour modifier les propriétés électroniques du matériau matriciel semi-conducteur en utilisant un ou plusieurs des composés de radialène selon la revendication 4.

12. Composant électronique selon la revendication 10 ou 11, sous la forme d'une diode électroluminescente organique, d'une cellule photovoltaïque, d'une cellule solaire organique, d'une diode organique ou d'un transistor à effet de champ organique.
